# FASCICULE DE BREVET EUROPEEN

(11) **EP 0 347 319 B1**
(45) Date de publication et mention de la délivrance du brevet: **07.01.1993**
(21) Numéro de dépôt: 89401673.2
(22) Date de dépôt: 15.06.1989
(51) Int. Cl.: A61F 13/15

(54) **Garniture absorbante**
Absorbierbare Vorlage
Absorbent article

(30) Priorité: 16.06.1988 FR 8808059
(43) Date de publication de la demande: 20.12.1989
(73) Titulaire: KAYSERSBERG SA, F-68240 Kaysersberg (FR)
(72) Inventeur: Marsot, Jacques, F-68000 Colmar (FR)
(74) Mandataire: David, Daniel

(56) Documents cités:
- EP-A- 0 270 058
- FR-A- 2 374 890
- US-A- 3 653 502
- US-A- 4 220 244
- US-A- 4 549 653
- US-A- 4 608 047

## Description

La présente invention concerne une garniture absorbante, notamment pour l'hygiène féminine.

Il est connu, notamment par la demande de brevet européen 012295 et par la demande de brevet français 2374890 d'entourer un article absorbant dans une feuille imperméable. Dans le cas de la demande de brevet français 2374890, la feuille imperméable entoure intégralement le tampon absorbant et une des grandes faces de l'article absorbant est munie de perforations. Dans ce cas, comme la feuille imperméable est située à l'extérieur de l'article, ce dernier doit être recouvert d'une feuille perméable souple en matériau textile ou non tissé, sur la face en contact avec le corps. Cette réalisation antérieure permet d'améliorer les capacités de rétention de l'article absorbant pour le transport et la présentation à la vente.

De même, le brevet européen 012295 enseigne une garniture absorbante entourée d'une enveloppe dont une couche interne est imperméable aux fuides, cette enveloppe présentant sur la face qui sera placée contre le corps une ouverture constituant une zone d'absorption. Cette enveloppe, dont une couche interne est imperméable, est utilisée pour améliorer la capacité d'absorption et le confort d'utilisation de l'article. Un tel article nécessite, malgré tout, pour sa distribution et son transport avant utilisation, un emballage pour protéger cet article d'hygiène.

Les demandes de brevets français 2548145 et 2494226 enseignent de tels emballages pour articles d'hygiène. Ces conditionnements ont la forme de pochette contenant un article d'hygiène replié de façon compacte. De tels conditionnements présentent l'inconvénient d'occasionner un gaspillage car l'enveloppe est ensuite jetée. De plus, les opérations de fabrication, nécessitant la reprise des articles absorbants et leur insertion dans le conditionnement en plus de la fabrication du conditionnement, occasionnent des frais supplémentaires. Par conséquent de tels articles conditionnés sont plus onéreux et plus complexes à fabriquer.

La présente invention a pour but de proposer une garniture absorbante qui constitue son propre conditionnement.

Ce but est atteint par le fait que la garniture absorbante comportant une bande de matériau absorbant formant un tampon d'absorption et de rétention de liquide, et une enveloppe extérieure entourant complétement le tampon est caractérisée en ce que ladite enveloppe constituée par exemple d'une feuille de matière plastique est imperméable aux liquides et l'une de ses faces est pourvue d'au moins une ligne de rupture, telle qu'une prédécoupe, selon un contour déterminé pour, après enlèvement de la surface de feuille imperméable intérieure à la ligne de rupture, libérer une zone de réception et d'absorption des fluides.

Selon une autre caractéristique de l'invention, les bords de la feuille imperméable, extérieurs aux contours de la ligne de rupture, sont rendus solidaires de la zone adjacente de la surface du tampon.

Selon une autre caractéristique, la face de l'enveloppe opposée à celle comportant la ligne de rupture comporte des moyens empêchant le glissement de la garniture par rapport au sous-vêtement.

Selon une autre caractéristique, l'enveloppe est pourvue de lignes de rupture de contour déterminé pour permettre à la fois le dégagement d'une zone de réception et la libération de volets latéraux, de fixation notamment.

D'autres caractéristiques et avantages de l'invention apparaîtront plus clairement à la lecture de la description ci-après, faite en référence aux dessins annexés sur lesquels :
- la Figure 1, représente une vue d'ensemble, en perspective avec coupe partielle, d'un premier mode de réalisation non limitatif de la garniture absorbante selon l'invention ;
- la Figure 2, représente une vue d'ensemble, en perspective avec coupe partielle, d'une deuxième réalisation de l'invention avant mise en service ;
- la Figure 3, représente la garniture de la figure 2, déployée, prête à l'emploi ;
- la Figure 4, représente une vue d'ensemble, en perspective avec coupe partielle, d'un troisième mode de réalisation à l'état déployé, la garniture étant prête à l'emploi ;
- la Figure 5, représente la garniture de la figure 4 avant sa mise en service.

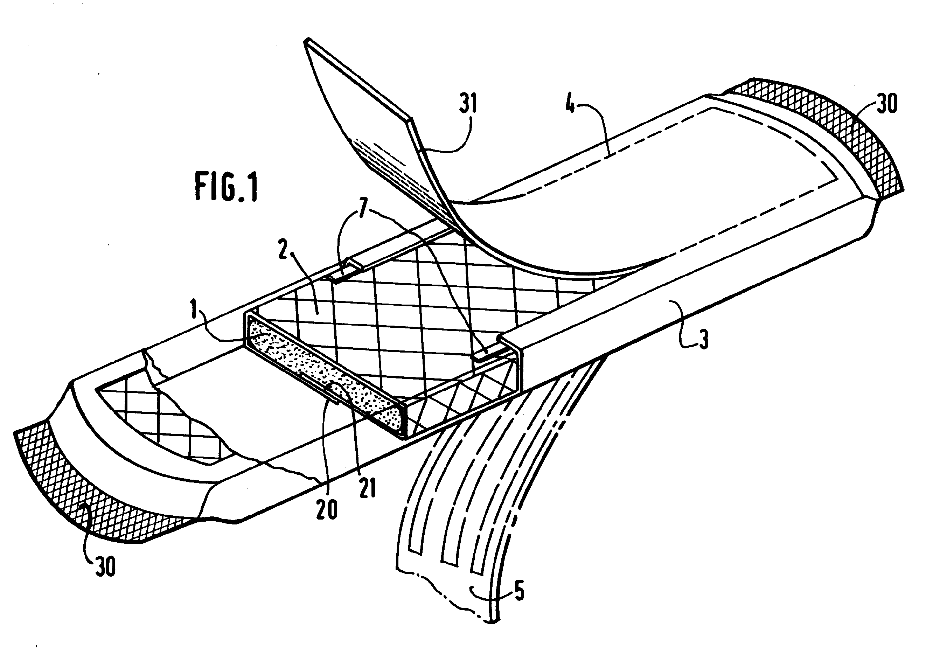

La garniture, selon un premier mode de réalisation de l'invention dont on voit le schéma à la figure 1, est constituée d'une bande de matériau absorbant (1), par exemple en mousse de cellulose. Cette bande (1) de dimension adéquate est enroulée dans une feuille (2) perméable en matériau non-tissé. Cette feuille (2) entoure la bande (1) sur toute sa longueur, et les bords (20, 21) de cette feuille (2) sont superposés et rendus solidaires sur la face inférieure de la garniture. Cette face inférieure est, comme on le verra plus tard, la face tournée vers le sous-vêtement de l'utilisateur. Le tampon d'absorption et de rétention des liquides, de forme générale parallélépipèdique, ainsi constitué par la bande (1) et la feuille (2) est enveloppé d'une feuille imperméable (3) en matière plastique, par exemple en polyéthylène. Cette enveloppe est fermée aux extrémités (30) de la garniture par une opération de thermosoudage. L'enveloppe (3) comporte, sur la face destinée à devenir la face supérieure de la garniture, face qui sera en contact avec le corps, une ligne de rupture (4) qui est dans le mode de réalisation représenté une prédécoupé de forme générale rectangulaire. Cette prédécoupé (4) délimite dans la feuille (3) une surface intérieure à la prédécoupé qui constitue une languette (31) que l'on peut déchirer pour permettre l'utilisation de la garniture. Comme on peut le voir sur la figure, les bords de la feuille (3) adjacents à la prédécoupé (4) et disposés à l'extérieur du contour de cette dernière sont rendus solidaires de la surface supérieure de la feuille (2) non-tissée, par exemple, par une bande de colle (7) disposée au moins le long des bords de la prédécoupé (4). La face inférieure de la serviette opposée à la face comportant la prédécoupé (4), comporte au moins une bande adhésive protégée par une bande de papier siliconé (5) que l'on enlève avant utilisation de la garniture.

Ainsi, on obtient de façon simple un emballage pour la garniture absorbante, emballage qui permet en même temps d'améliorer les capacités de rétention de l'article absorbant et son confort d'utilisation. En effet, lors de l'utilisation, la languette (31) étant enlevée, la feuille imperméable (3) évitera au liquide de s'écouler vers le bas ou sur les côtés.

Il est bien évident que la découpe (4) de forme rectangulaire peut avoir toute autre forme, plus adaptée à l'utilisation. De même, la garniture peut en particulier comporter des rétrécissements de sa largeur, par exemple dans la zone centrale. La bande constituant le tampon rectangulaire peut également avoir une section en forme de trapèze, au lieu d'avoir une section rectangulaire, de façon à donner à l'article une forme plus adaptée à l'anatomie du sujet utilisateur.

Selon une variante non représentée, la feuille imperméable se compose d'au moins deux éléments réunis par soudage ou collage ; par exemple un élément supérieur et un élément inférieur. Pour dégager la zone d'absorption, on détache l'élément approprié suivant par exemple une ligne de rupture constituée le long de la zone de liaison entre les deux éléments. Le voile perméable peut éventuellement ne recouvrir que la partie du tampon absorbant correspondant à la zone d'absorption.

Selon un deuxième mode de réalisation, la garniture absorbante comporte des volets latéraux dont la fonction est d'assurer un maintien au sous-vêtement et une protection améliorés, comme cela est connu en soi.

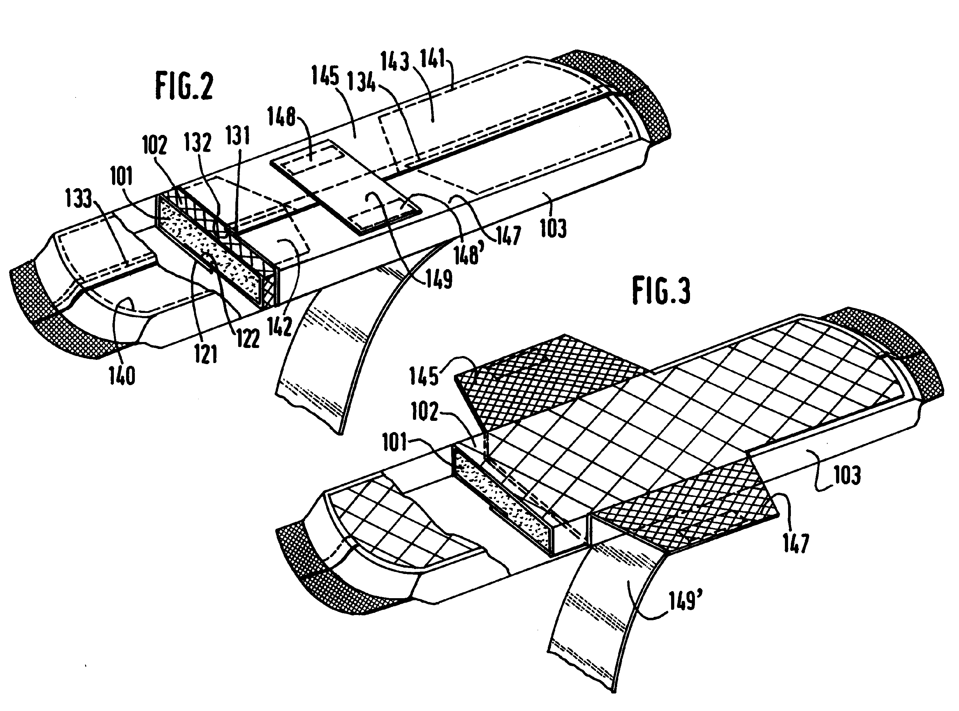

La figure 2 représente une telle garniture constituant, conformément à l'invention, son propre conditionnement.

Elle comprend un tampon absorbant (101) de forme oblongue, parallélépipèdique ou autre, en ouate de cellulose par exemple, enveloppé dans un voile de non-tissé perméable (102) sur toute sa longueur. Les bords (121) et (122) du voile (102) sont superposés et rendus solidaires par collage ou thermoscellage sur la face inférieure de la garniture, c'est-à-dire tournée du côté opposé à celle qui reçoit les fluides corporels.

L'ensemble formé par le tampon d'absorption et le voile perméable est lui-même enveloppé dans une feuille imperméable (103) en matière plastique souple telle que le polyéthylène.

Dans ce mode de réalisation, les bords longitudinaux (131) et (132) de la feuille (103) se superposent sur la face supérieure, c'est-à-dire la grande face tournée du côté destiné à recevoir les fluides, et sont solidarisés au moyen de deux traits de colle (133) et (134), ou de soudure, dans le prolongement l'un de l'autre. L'enveloppe est fermée à ses deux extrémités longitudinales par thermoscellage des bords. Le non-tissé est rendu solidaire de l'enveloppe imperméable le long des bords longitudinaux de la face supérieure par collage ou thermosoudage. Comme on le voit sur la figure, la face supérieure de l'enveloppe (103) comprend une ligne de rupture en deux parties (140) et (141). Il peut s'agir par exemple d'une prédécoupé obtenue par découpage du matériau en traits interrompus. Ces deux lignes de rupture, fermées, délimitent deux languettes (142) et (143) espacées l'une de l'autre dans le sens de la longueur.

Les deux languettes sont séparées par une zone formée par les deux volets (145) et (147) proprement dits. Pour chaque languette la ligne de rupture, (140) et (141) respectivement, longe le bord transversal, les bords longitudinaux en partie et les volets (145) et (147).

Les bords de l'enveloppe dans la zone des volets ne sont pas scellés et correspondent à l'interruption des deux traits de soudure (133) et (134).

Chacun des volets est pourvu d'un moyen d'attache (148) et (148′), qui peut être obtenu au moyen d'une enduction de colle dite "hot-melt" présentant un tack à froid. Un papier siliconé (149) protège la colle avant emploi.

Sur la face opposée, la garniture est pourvue d'un moyen d'attache, de préférence une enduction de colle également protégée par un papier siliconé (149′) avant emploi.

Pour la mise en service de la garniture, on détache les deux languettes (142) et (143) le long de leur ligne de rupture respective, et on déploie les deux volets en les faisant pivoter autour d'un axe longitudinal, après avoir oté le papier siliconé (149).

On découvre ainsi, voir figure 3, la face supérieure du tampon, revêtue du non-tissé perméable, pour la réception des fluides corporels.

On peut ensuite mettre la garniture en place dans le sous-vêtement en appliquant la face inférieure contre le fond de ce dernier, et en rabattant les deux volets autour de la lisière pour les fixer sur la face du sous-vêtement tournée vers l'extérieur.

Afin d'améliorer le toucher et la protection, on peut prévoir de recouvrir au moins la face interne des volets par un non-tissé ou un matériau absorbant.

Dans un troisième mode de réalisation, le tampon absorbant n'est pas enveloppé entièrement dans le non-tissé. Celui-ci revêt seulement la face de réception des fluides.

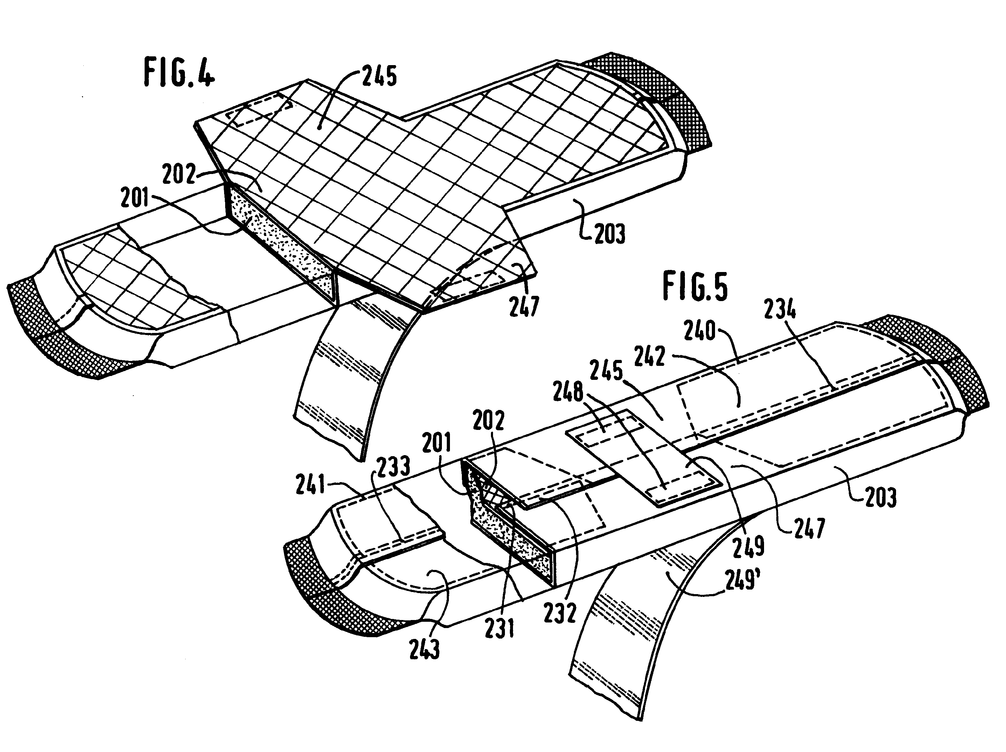

Sur la figure 4, on a représenté une telle garniture. Le tampon (201) est enserré entre le voile perméable (202) recouvrant la grande face supérieure et la feuille imperméable (203) recouvrant la grande face inférieure.

Le voile et la feuille ont été rendus solidaires l'un à l'autre par collage ou thermosoudage le long des bords du tampon de façon à l'enfermer complètement.

Le voile perméable et la feuille imperméable se prolongent en deux volets latéraux (245) et (247) dans la zone médiane de la garniture.

La figure 5 représente la garniture avant sa mise en service, où la feuille imperméable, conformément à l'invention, enveloppe entièrement le tampon pour former son emballage.

La feuille (203) se prolonge sur la face supérieure du tampon avec chevauchement des deux bords longitudinaux (231) et (232).

Comme dans la réalisation précédente, deux languettes (242) et (243) sont ménagées sur cette partie de l'enveloppe par deux lignes de rupture fermées (240) et (241). Pour chaque languette, la ligne de rupture longe un bord transversal de la face supérieure du tampon, et une partie des bords longitudinaux jusqu'à la zone définissant les volets (245) et (247) dont elle suit un bord.

Dans la région de chevauchement située entre les languettes, les bords de la feuille (203) ne sont pas scellés ou bien peuvent être désolidarisés aisément quand on exerce une traction sur eux.

Les deux extrémités de la garniture sont thermoscellés.

Les volets (245) et (247) sont pourvus de moyens d'attache adhésive au sous-vêtement (248) protégés par une bande de papier siliconé (249).

Comme précédemment également, la face inférieure comporte des moyens d'attache adhésive protégés par une bande de papier siliconé (249′).

La mise en service de la garniture est obtenue de la même façon que dans le mode de réalisation précédente.

Cette variante où tampon est compris entre un voile perméable et une feuille imperméable peut être étendue au premier mode de réalisation sans volets latéraux.

## Revendications

1. Garniture absorbante comportant une bande de matériau absorbant (1, 101, 201) formant un tampon d'absorption et de rétention des liquides et une enveloppe extérieure (3, 103, 203) entourant complètement le tampon, dans laquelle ladite enveloppe, constituée par exemple d'une feuille de matière plastique, est imperméable aux liquides, caractérisée en ce que l'une des grandes faces de ladite enveloppe est pourvue d'au moins d'une ligne de rupture (4 ; 140, 141 ; 240, 241), telle qu'une prédécoupe, selon un contour déterminé pour, après enlèvement de la surface (31 ; 142, 143 ; 242, 243) de feuille imperméable intérieure à la ligne de rupture (4 ; 140, 141 ; 240, 241) libérer une zone de réception et d'absorption et en ce que les bords de la feuille (3) imperméable, extérieurs aux contours de la ligne de rupture (4), sont rendus solidaires de la zone adjacente (2) de la surface du tampon.

2. Garniture selon la revendication 1 caractérisée en ce que la face de l'enveloppe (3) opposée à celle comportant la prédécoupe (4) comporte des moyens empêchant le glissement de la garniture par rapport au sous-vêtement.

3. Garniture absorbante selon l'une des revendications précédentes caractérisée en ce que la bande de matériau (1, 101) absorbant est entourée d'un voile en matériau non tissé (2, 102).

4. Garniture absorbante selon l'une des revendications 1 à 3 caractérisée en ce que la bande de matériau absorbant (201) est comprise entre un voile non tissé (202) recouvrant une de ses grandes faces et une feuille imperméable (203) recouvrant la grande face opposée.

5. Garniture absorbante selon l'une des revendications précédentes, caractérisée en ce que la feuille imperméable se compose d'au moins deux éléments, réunis au moins partiellement suivant ladite ligne de rupture.

6. Garniture absorbante selon l'une des revendications précédentes caractérisée en ce que l'enveloppe est pourvue de deux lignes de rupture (140, 141 ; 240, 241) selon un contour déterminé pour après enlèvement des surfaces (142, 143 ; 242, 243) de feuille imperméable intérieures auxdites lignes de rupture, dégager une zone pour la réception des fluides et libérer des volets latéraux (145, 147 ; 245, 247) destinés notamment à la fixation de la garniture au sous-vêtement.

7. Garniture absorbante selon la revendication précédente, dont la feuille imperméable comporte deux bords latéraux (131, 132 ;) qui sont rendus solidaires pour la formation de l'enveloppe caractérisée en ce que lesdits bords latéraux se rejoignent sur la face de la garniture située du côté de réception de fluides, la partie des bords latéraux coïncidant avec les volets (145, 147 ; 245, 247) étant susceptible de se désolidariser pour permettre la libération desdits volets.

8. Garniture absorbante selon l'une des revendications précédentes caractérisée en ce que la feuille imperméable (103, 203) est revêtue, au moins dans la région constituant les volets latéraux (145, 147 ; 245, 247), d'un voile non tissé ou d'une matière absorbante.

## Claims

1. Absorbent towel, comprising a strip of absorbent material (1, 101, 201) forming a tampon for absorbing and retaining liquids, and an external sheath (3, 103, 203) completely surrounding the tampon, in which the said sheath, consisting for example of a sheet of plastic, is impermeable to liquids, characterised in that one of the large faces of the said sheath is provided with at least one breaking line (4, 140, 141; 240, 241), such as a perforation, in accordance with an outline determined so that, after the surface (31; 142, 143; 242, 243) of the impermeable sheet within the breaking line (4; 140, 141; 240, 241) is removed, an area for receiving and absorbing will be revealed, and in that the edges of the impermeable sheet (3), outside the contours of the breaking line (4), are attached to the adjacent area (2) of the surface of the tampon.

2. Towel according to Claim 1, characterised in that the face of the sheath (3) opposite to the one incorporating the perforation (4) incorporates means for preventing the towel from sliding in relation to the undergarment.

3. Absorbent towel according to one of the preceding claims, characterised in that the strip of absorbent-material (1, 101) is surrounded by a non-woven material (2, 102).

4. Absorbent towel according to one of Claims 1 to 3, characterised in that the strip of absorbent material (201) is enclosed between a non-woven material (202) covering one of its large faces and an impermeable sheet (203) covering the opposite large face.

5. Absorbent towel according to one of the preceding claims, characterised in that the impermeable sheet consists of at least two components, joined at least partially along the said breaking line.

6. Absorbent towel according to one of the preceding claims, characterised in that the sheath has two breaking lines (140, 141; 240, 241) along a given contour so as to expose, after the surfaces (142, 143; 242, 243) of impermeable sheet within the said breaking lines have been removed, an area for receiving fluids, and to release lateral flaps (145, 147; 245, 247) intended notably for fixing the towel to the undergarment.

7. Absorbent towel according to the preceding claim, in which the impermeable sheet has two lateral edges (131, 132;) which are joined together to form the sheath, characterised in that the said lateral edges join on the face of the towel situated on the side where fluids are received, it being possible to detach the parts of the lateral edges coinciding with the flaps (145, 147; 245, 247) so as to allow the said flaps to be released.

8. Absorbent towel according to one of the preceding claims, characterised in that the impermeable sheet (103, 203) is covered, at least in the area constituting the lateral flaps (145, 147; 245, 247), with a non-woven or absorbent material.

## Patentansprüche

1. Absorbierende Vorlage, mit einem Streifen aus absorbierendem Material (1, 101, 201), der ein Kissen zum Absorbieren und Zurückhalten von Flüssigkeit bildet und mit einer äußeren Hülle (3, 103, 203), die das Kissen vollständig umgibt, worin die Hülle, die beispielsweise von einer Folie aus Kunststoff gebildet ist, für Flüssigkeiten undurchlässig ist, dadurch gekennzeichnet, daß eine der großen Seiten der Hülle entlang einer bestimmten Kontur mit zumindest einer Aufreißlinie (4; 140, 141; 240, 241), wie mit einem Voreinschnitt, versehen ist, um nach dem Entfernen der Außenseite (31; 142, 143; 242, 243) der undurchlässigen Schicht innerhalb der Aufreißlinie (4; 140, 141; 240, 241) eine Zone für die Aufnahme und Absorption freizugeben, und daß die Ränder der undurchlässigen Folie (3) außerhalb der Konturen der Aufreißlinie (4) an der benachbarten Zone (2) der Oberfläche des Kissens festgelegt sind.

2. Vorlage nach Anspruch 1, dadurch gekennzeichnet, daß die Seite der Hülle (3) gegenüber derjenigen, die den Voreinschnitt (4) aufweist, Mittel hat, die ein Verrutschen der Vorlage bezüglich der Unterwäsche verhindern.

3. Absorbierende Vorlage gemäß einem der vorstehenden Ansprüche, dadurch gekennzeichnet, daß der Streifen aus absorbierendem Material (1, 101) von einer Umhüllung aus einem Nonwoven (2, 102) umgeben ist.

4. Absorbierende Vorlage gemäß einem der Ansprüche 1 bis 3, dadurch gekennzeichnet, daß das Kissen aus absorbierendem Material (201) in einer Abdeckung aus einem Nonwoven (202), die eine seiner großen Seiten überdeckt, und einer undurchlässigen Folie (203), welche die gegenüberliegende Seite überdeckt, enthalten ist.

5. Absorbierende Vorlage gemäß einem der vorstehenden Ansprüche, dadurch gekennzeichnet, daß sich die undurchlässige Folie aus zumindest zwei Elementen zusammensetzt, die zumindest teilweise entlang der Aufreißlinie verbunden sind.

6. Absorbierende Vorlage gemäß einem der vorstehenden Ansprüche, dadurch gekennzeichnet, daß die Umhüllung mit zwei Aufreißlinien (140, 141; 240, 241) entlang einer vorbestimmten Kontur versehen ist, um nach dem Entfernen der Außenflächen (142, 143; 242, 243) undurchlässiger Folie innerhalb der Aufreißlinien eine Zone für die Aufnahme von Flüssigkeiten freizusetzen und seitliche Flügel (145, 147; 245, 247) freizugeben, die insbesondere der Befestigung der Vorlage an der Unterwäsche dienen.

7. Absorbierende Vorlage gemäß vorstehendem Anspruch, wovon die undurchlässige Folie zwei seitliche Ränder (131, 132) aufweist, die zum Bilden einer Umhüllung miteinander verbunden sind, dadurch gekennzeichnet, daß die Seitenränder auf der Seite der Folie zusammentreffen, die auf der Seite der Aufnahme von Flüssigkeiten angeordnet ist, wobei der mit den Flügeln (145, 147; 245, 247) zusammentreffende Teil der Seitenränder imstande ist, sich zu lösen, um eine Freigabe der Flügel zu ermöglichen.

8. Absorbierende Vorlage gemäß einem der vorstehenden Ansprüche, dadurch gekennzeichnet, daß die undurchlässige Folie (103, 203) zumindest in dem Bereich, der die seitlichen Flügel (145, 147; 245, 247) bildet, von einer Umhüllung aus Nonwoven oder absorbierendem Material überzogen ist.
